# EUROPEAN PATENT SPECIFICATION

(11) **EP 3 878 476 B1**
(45) Date of publication and mention of the grant of the patent: **01.10.2025**
(21) Application number: 19882937.6
(22) Date of filing: 01.11.2019
(51) Int. Cl.: A61K 49/18, A61K 9/06, A61K 47/36, A61K 45/06, A61B 90/00

(54) **ALGINIC ACID-BASED INJECTABLE HYDROGEL SYSTEM**
INJIZIERBARE HYDROGELSYSTEME AUF DER BASIS VON ALGINSÄURE
SYSTÈME D'HYDROGEL INJECTABLE À BASE D'ACIDE ALGINIQUE

(30) Priority: 06.11.2018 KR 20180135161
(43) Date of publication of application: 15.09.2021
(73) Proprietor: National Cancer Center, Goyang-si, Gyeonggi-do 10408 (KR)
(72) Inventor: CHOI, Yong Doo, Goyang-si Gyeonggi-do 10408 (KR); LEE, Seon Sook, Seoul 05748 (KR); KIM, Hyun Jin, Goyang-si Gyeonggi-do 10407 (KR); SOHN, Dae Kyung, Seoul 06518 (KR); KIM, Seok Ki, Goyang-si Gyeonggi-do 10408 (KR); KIM, Young Woo, Goyang-si Gyeonggi-do 10408 (KR); RYU, Keun Won, Goyang-si Gyeonggi-do 10408 (KR); YOON, Hong Man, Goyang-si Gyeonggi-do 10408 (KR)
(74) Representative: Algemeen Octrooi- en Merkenbureau B.V.
(86) International application number: PCT/KR2019/014682
(87) International publication number: WO 2020/096281

(56) References cited:
- EP-A2- 1 799 232
- WO-A2-2006/044342
- WO-A2-2012/012772
- KR-A- 20060 021 967
- US-A1- 2016 303 291
- LARSEN, B. E.: "Rheological characterization of an injectable alginate gel system", BMC BIOTECHNOLOGY, 2015, XP021221192
- HIGHAM, A. K.: "Photo-activated ionic gelation of alginate hydrogel: real-time rheological monitoring of the two-step crosslinking mechanism.", SOFT MATTER, 2014, XP055704964

## Description

### [Technical Field]

The present disclosure relates to an alginic acid-based injectable hydrogel system as described in the present claims, and in particular, to an alginic acid-based injectable hydrogel system which may help to stay at the injection site for a certain period of time to accurately determine a position of a target site by an image, without diffusing a contrast agent contained in an alginic acid to the surroundings and thereby helps to treat or alleviate symptoms at the target site by slowly releasing the drug contained in alginic acid at the target site.

### [Background Art]

As one of the 'minimal invasive surgeries' which reduces the injury or influence on the patient's body, laparoscopic surgery is a surgery mode using surgical tools (forceps, electric scalpel, hemostasis suture, etc.) while puncturing a small hole of 0.5 to 1.5 cm and inserting endoscope (laparoscope) loaded with a special camera into the body, looking inside the abdominal cavity, instead of incising the abdomen or chest.

Laparoscopic surgery has been advanced over the past several decades with the advantages of faster recovery and shorter hospital stays as it has fewer postoperative pain and fewer incisions than a laparotomy. Recently, as robotic surgery using this laparoscopic surgery has been introduced, laparoscopic surgery has been accepted as the basis of surgical techniques. For the surgical treatment of colorectal cancer, gastric cancer, etc., a process of visually confirming the treatment site, such as the cancer site by inserting an endoscope into the stomach or the large intestine before incision and then removal of the cancer lesion in the body through laparoscopic surgery is needed. However, since the viewing angle and range obtained through the endoscope and the viewing angle and range obtained through the laparoscope are completely different in the surgery of prior art, it is difficult to accurately find the target position to be incised, and thus the accuracy and the range of the incision are inevitably different depending on the skill of the operator, and the range to be incised is inevitably increased. Therefore, in order to label the region to be removed by surgery, a method of injecting a drug such as Indian ink into the lesion identified through an endoscopic injection needle with the endoscope to label the lesion, and then performing surgery for the region labeled with Indian ink is used, while checking with the naked eye through laparoscopy at the time of surgery. However, such a labeling method has a disadvantage that complications may occur due to ink incorrectly injected into the muscle layer, and that the region to be removed by surgery is widened as ink or dye spreads around the injected area. Therefore, in order to minimize the region from which normal tissue is removed during laparoscopic surgery, a contrast agent for "marking" or "marker" is required to label an appropriate disease lesion location.

If surgical treatment is difficult for a region of a cancer or inflammatory disease, treatment and symptom relief may be achieved by injecting a drug locally, and in the case of hydrophobic drugs, since they are not easily soluble in water, local injection of the drugs is not easy and in the case of hydrophilic drugs, since they spread quickly at the injected site, the duration of the drug is very short. Therefore, there is a need for a "locally injectable" drug formulation capable of maximizing therapeutic effect or symptom relief effect at the target site by allowing the drug to be released slowly at a desired rate while allowing the drug to be evenly injected into the target lesion.

US 2016/030291 A1 describes pharmaceutical formulations of homogenous hydrogels comprising FGF-18 and method for producing such, and their use in the treatment of cartilage disorders. WO 2006/044342 (also published as EP1799232 A2) describes kits and compositions for producing an alginate gel, and implantable devices comprising such gels. KR 2006 0021967 describes water insoluble alginate gel for delivering tooth whitening agents.

### [Disclosure]

### [Technical Problem]

There may be various image contrast agent or image methods capable of labeling lesions, and recently, a technique for labeling lesions using near-infrared fluorescent dyes is rapidly developing. Fluorescence image technology using near-infrared fluorescent dyes, has the advantage of not only being able to detect even a labeled region located deep in a tissue from the fluorescent image, but also sensitively detecting a fluorescent signal even with a small amount of fluorescent dye. As a conventional fluorescent image contrast agent, indocyanine green, a near-infrared fluorescent dye that has been clinically approved by the FDA, has been used for image diagnosis and vascular image during surgery, but there are several limitations in its clinical application. Since there is a disadvantage that Indocyanine green decreases fluorescence brightness due to concentration-dependent aggregation phenomenon, has chemical instability in aqueous solution in vivo, and spreads rapidly to surrounding tissues to reduce image discrimination accuracy and rapidly weaken the image signal intensity, it makes the contrast agent difficult to be used. For this reason, there is a need to develop a method that can increase the labeling efficiency for lesions and increase the halflife of the contrast agent in vivo.

Next, when the drugs are injected into a living body, side effects may occur on the surrounding normal tissues by being rapidly absorbed and spreading at the site where the drug was injected, and since the drugs are decomposed by enzyme action in the living body, resulting in poor drug persistence. Thus, there is a difficulty in administering large amounts of drugs or repeatedly administering drugs in order to obtain a desired drug effect. For this reason, there is a need to develop a method capable of improving the duration of the drug effect on the target site by preventing the diffusion of the drug in vivo.

The present disclosure was conceived to solve the problems of the prior art as described above, and the present inventors have completed the invention by developing an alginic acid-based injectable hydrogel system using polyvalent ions as an ionic cross-linking agent based on alginic acid. Accordingly, the present disclosure provides a composition for preparing an injectable hydrogel, comprising a first agent comprising alginic acid and an ionic crosslinking agent; and a second agent comprising alginic acid and a crosslinking rate controller. In addition, the present disclosure provides a composition for labeling the lesion, comprising a first agent comprising alginic acid and an ionic crosslinking agent; and a second agent comprising alginic acid and a crosslinking rate controller, and a method for labeling the lesion, comprising injecting the composition to an individual.

An alginic acid-based injectable hydrogel system according to the present disclosure allows the contrast agent contained in alginic acid to stay at the injection site for a certain period of time without being diffused to the surroundings, so that the location of the target site may be accurately determined by image.

In addition, the alginic acid-based injectable hydrogel system according to the present disclosure may include a drug as well as a contrast agent into alginic acid and inject them into the target site, and in particular, if the alginic acid hydrogel according to the present disclosure including a drug such as an anticancer agent is injected to the target site, the drug may be released slowly for a long period of time from alginic acid while preventing the drug from spreading to the surroundings, so that the effect of drugs can last for a long time.

### [Technical Solution]

Unless otherwise defined, all technical and scientific terms used in this specification have the same meaning as commonly understood by an expert skilled in the art to which the present disclosure belongs. In general, the nomenclature used in this specification is well known and commonly used in the art.

In a first aspect the invention describes a composition for preparing an injectable hydrogel, comprising a first agent comprising aqueous alginic acid and an ionic crosslinking agent; and a second agent comprising aqueous alginic acid and a crosslinking rate controller, wherein the crosslinking rate controller comprises any of one or more selected from the group consisting of D-gluconic acid, acetic acid, malic acid, lactic acid and ascorbic acid. In a second aspect the invention further describes the composition according to the first aspect of the invention for use in surgery. In a third aspect the invention further describes the composition according to the first aspect of the invention for use as a medicament, wherein the composition comprises a drug.

The present disclosure relates to an alginic acid-based injectable hydrogel system to address the above-described technical problem. Specifically, the present disclosure provides an alginic acid-based injectable hydrogel system in the form of a composition for preparing an injectable hydrogel, comprising a first agent comprising alginic acid and an ionic crosslinking agent; and a second agent comprising alginic acid and a crosslinking rate controller. In addition, the present disclosure provides a composition for labeling the lesion, comprising a first agent comprising alginic acid and an ionic crosslinking agent; and a second agent comprising alginic acid and a crosslinking rate controller, and a method for labeling the lesion, comprising injecting said composition to an individual.

In the present disclosure, the first agent or the second agent may comprise an image contrast agent or a drug.

That is, the composition for preparing an injectable hydrogel according to the present disclosure was made into an injectable hydrogel form by mixing the first and second agents and injecting the mixture, in which an image contrast agent or a drug is incorporated into an alginic acid or alginic acid/hyaluronic acid mixture, and an ionic crosslinking agent and a crosslinking rate controller are included therein.

Accordingly, the present disclosure relates to an injectable hydrogel composition in which the first agent and the second agent are mixed together.

When an image contrast agent or a drug is topically injected into the target lesion region using such an alginic acid-based injectable hydrogel system, the injection solution becomes a gel at the labeling site, thereby preventing the contrast agent or drug from spreading, and resulting in increasing the duration. Through this, not only can the location of the target site be determined relatively accurately by image, but also in the case of a drug, the drug effect can last for a long time, so that side effects caused by the diffusion of the drug may be reduced, and the desired drug effect may be achieved at the target site.

In the present disclosure, the "ionic crosslinking agent" is included in the first agent and serves as an ion source that provides polyvalent ions for cross-linking of alginic acid to form the ionic cross-linked hydrogel. These ionic crosslinking agents may be selected from the group consisting of calcium carbonate, strontium carbonate, calcium disodium edetate (calcium EDTA), calcium citrate, calcium sulfate, calcium alginate, calcium gluconate, calcium phosphate dibasic (CaHPO₄), barium carbonate, zinc carbonate, calcium chloride, calcium lactate, calcium aspartate, calcium saccharate, calcium oxovalerate, calcium lactobionate, calcium lactogluoconate, and a mixture thereof, but are not limited thereto.

In the present disclosure, the "crosslinking rate controller" is included in the second agent and serves to control the rate of alginic acid cross-linking formation. Such a crosslinking rate controller may comprise any one or more selected from the group consisting of an organic acid such as gluconic acid, acetic acid, malic acid, lactic acid, ascorbic acid, or a salt thereof or a mixture thereof, and a photoacid generator (PAG). More specifically, the crosslinking rate controller may be D-gluconic acid, but is not particularly limited thereto.

In the present disclosure, the ratio of an ionic crosslinking agent and a crosslinking rate controller is not limited, but the ionic crosslinking agent is preferable to have a molar ratio of 0.1 to 0.4X based on the number of moles (X) of a carboxylic acid group present in alginic acid. In the case of the crosslinking rate controller, it is used in an amount that makes the pH of the solution neutral.

In the present disclosure, the alginic acid may be mixed with hyaluronic acid. Since the hydrogel is well formed even under various mixing conditions in which the mixing ratio of alginic acid and hyaluronic acid is from 100:0 to 1:99, the mixing ratio of alginic acid and hyaluronic acid is not significantly limited. Hyaluronic acid is decomposed by hyaluronidase, which is an enzyme present in the human body, so the higher the mixing ratio of hyaluronic acid, the faster the decomposition rate of the hydrogel and the faster the release rate of the drug, and the mixing ratio of alginic acid and hyaluronic acid is adjusted according to the desired release rate of the contrast agent or drug.

In the present disclosure, the "image contrast agent" may be a magnetic resonance image contrast agent, a computed tomography (CT) contrast agent, or a fluorescent dye.

When the magnetic resonance image contrast agent (MRI contrast agent) is injected into the human body, it changes the relaxation rate of tissues to widen the difference in relaxation degree between tissues, and induces change in MRI signal to make the contrast between tissues more clearly. The MRI contrast agent includes ionized gadolinium (Gd) (III) complexes and neutral gadolinium (Gd) (III) complexes. More specifically, the MRI contrast agent may be a gadolinium complex, a manganese complex, a copper (II) complex, an iron oxide nanoparticle, and manganese oxide nanoparticles. In an embodiment of the present disclosure, a manganese oxide nanoparticle, and Feridex^{™}, which is iron oxide nanoparticle contrast agent, and manganese chloride were used as MRI contrast agent, but are not limited thereto.

In the present disclosure, the CT contrast agent as a material used to clarify shading when X-rays are taken, may be made of a compound containing a metal or iodine, or nanoparticles, and Rose Bengal is used in an embodiment of the present disclosure, but is not limited thereto.

In the present disclosure, the "fluorescent dye" may be fluorescein, CR110: carboxyrhodamine 110: rhodamine green (trade name), TAMRA: carboxytetramethylrhodamine: TMR, carboxyrhodamine 6G: CR6G, BODIPY FL (trade name): 4,4-difluoro-5,7-dimethyl-4-bora-3a,4a-diaza-s-indacene-3-propionic acid, BODIPY 493/503 (trade name): 4,4-difluoro-1,3,5,7-tetramethyl-4-bora-3a,4a-diaza-s-indacene-8-propionic acid, BODIPY R6G (trade name): 4,4-difluoro-5-(4-phenyl-1,3-butadienyl)-4-bora-3a,4a-diaza-s-indacene-3-propionic acid, BODIPY 558/568 (trade name): 4,4-difluoro-5-(2-thienyl)-4-bora-3a,4a-diaza-s-indacene-3-propionic acid, BODIPY 564/570 (trade name): 4,4-difluoro-5-styryl-4-bora-3a,4a-diaza-s-indacene-3-propionic acid, BODIPY 576/589 (trade name): 4,4-difluoro-5-(2-pyrrolyl)-4-bora-3a,4a-diaza-s-indacene-3-propionic acid, BODIPY 581/591 (trade name): 4,4-difluoro-5-(4-phenyl-1,3-butadienyl)-4-bora-3a,4a-diaza-s-indacene-3-propionic acid, EvoBlue10 (trade name), EvoBlue30 (trade name), MR121, ATTO 655 (trade name), ATTO 680 (trade name), ATTO 700 (trade name), ATTO MB2 (trade name), Alexa Fluor 350 (trade name), Alexa Fluor 405 (trade name), Alexa Fluor 430 (trade name), Alexa Fluor 488 (trade name), Alexa Fluor 532 (trade name), Alexa Fluor 546 (trade name), Alexa Fluor 555 (trade name), Alexa Fluor 568 (trade Name), Alexa Fluor 594 (trade name), Alexa Fluor 633 (trade Name), Alexa Fluor 680 (trade name), Alexa Fluor 700 (trade name), Alexa Fluor 750 (trade name), Alexa Fluor 790 (trade name), Flamma 496 (trade name), Flamma 507 (trade name), Flamma 530 (trade name), Flamma 552 (trade name), Flamma 560 (trade name), Flamma 575 (trade name), Flamma 581 (trade name), Flamma 648 (trade name), Flamma 675 (trade name), Flamma 749 (trade name), Flamma 774 (trade name), Flamma 775 (trade name), Rhodamine Red-X (trade name), Texas Red-X (trade name), 5(6)-TAMRA-X (trade name), 5TAMRA (trade name), cyanine-based dyes (Cy5, Cy5.5, Cy7, IR820), Indocyanine green (ICG) or ZW800, etc., but is not limited thereto. Specifically, the fluorescent dye may be a complex in which a fluorescent dye and human serum albumin are bound. The bond may be an ionic bond, a hydrophobic bond, or a covalent bond, and more specifically, a covalent bond, but is not particularly limited thereto. Typically, a BODIPY dye may form a hydrophobic bond with human serum albumin, and cyanine-based dyes and Alexa-based dyes introduced with a sulfonyl group to improve hydrophilicity may form ionic bonds.

In the present disclosure, the "drug" is not particularly limited in its kind. A drug suitable for the target disease which may be selected, may be for example, anticancer agents, anti-inflammatory agents, anesthetic agents, antiviral agents, antibacterial agents, therapeutic antibodies, antibiotics, or immunotherapeutic agents, etc.

In the present disclosure, examples of the anticancer agent may include, but are not limited to, one or more selected from the group consisting of doxorubicin, gosipol, nitrogen mustard, imatinib, oxaliplatin, rituximab, erlotinib, neratinib, lapatinib, gefitinib, vandetanib, nitrotinib, semasanib, bosutinib, akcitinib, cediranib, restaurtinib, trastuzumab, gefitinib, bortezomib, sunitinib, carboplatin, bevacizumab, cisplatin, cetuximab, viscumalbum, asparaginase, tretinoin, hydroxycarbamide, dasatinib, estramustine, gemtuzumabozogamycin, ibritumomab tiuxetan, heptaplatin, methylaminolevulinic acid, amsacrine, alemtuzumab, procarbazine, alprostadil, holmium nitrate chitosan, gemcitabine, doxyfluridine, pemetrexed, tegafur, capecitabine, gimeracin, oteracil, azacitidine, methotrexate, uracil, cytarabine, fluorouracil, fludarabine, enocitabine, flutamide, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleromycin, daunorubicin, dactinomycin, pyrarubicin, aclarubicin, pepromycin, temsirolimus, temozolomide, busulfan, ifosfamide, cyclophosphamide, melpharan, altrethamine, dacarbazine, thiotepa, nimustine, chlorambucil, mitolactol, leucovorin , tretonin, exmestane, aminoglutesimide, anagrelide, navelbin, padrazole, tamoxifen, toremifen, testolactone, anastrozole, letrozole, borozole, bicalutamide, lomustine and carmustine.

In the present disclosure, examples of the anti-inflammatory agent may include salicylates, ibuprofen, naproxen, fenoprofen, indomethacin, phenyltazone, methotrexate, cyclophosphamide, mechlorethamine, dexamethasone, prednisolone, celecoxib, valdecoxib, nimesulide, cortisone, or corticosteroid.

On the other hand, when an alginic acid-based hydrogel is injected into the lower mucosa for image labeling, the patient's immune system recognizes that a foreign substance has invaded into the living body and may cause an inflammatory reaction in removing the foreign substance. At this time, proteins including various enzymes, complement systems, antibodies, and immune cells are introduced into the injection site, and actions to remove alginic acid and fluorescent dyes present at the injection site occur. Therefore, there is a possibility that the rate at which the fluorescent dye contained in the hydrogel is removed due to the inflammatory reaction occurring at the injection site would be fast. As a method for reducing the inflammatory reaction and side effects, there is a method of separately orally administering an anti-inflammatory agent or injecting an anti-inflammatory agent into a target site by mixing with a hydration gel, when injecting a hydration gel. Since hydrophobic anti-inflammatory drugs do not dissolve well in water, they are loaded into microspheres, nanoparticles, liposomes or micelles, etc., to ensure good dispersion in aqueous solution and injected with a hydrogel.

The composition for preparing the injectable hydrogel according to the present disclosure may contain a suspending agent, a solubility aid, a stabilizer, an isotonic agent, a preservative, an adsorption inhibitor, a surfactant, a diluent, an excipient, a pH adjuster, a painless agent, a buffer, and a sulfur-containing reducing agent, antioxidant, etc., as appropriate, if necessary. For example, sterile water, physiological saline, conventional buffers (phosphoric acid, citric acid, other organic acids, etc.), stabilizers, salts, antioxidants (ascorbic acid, etc.), surfactants, suspending agents, isotonic agents, or preservatives, etc., may be included. As an aqueous solution for injection, for example, an isotonic solution including physiological saline, glucose or other adjuvants, such as D-sorbitol, D-mannose, D-mannitol, sodium chloride, and also buffers, for example, a phosphate buffer solution, a sodium acetate buffer solution, a painless agent such as procaine hydrochloride, a stabilizer such as benzyl alcohol, phenol, or an antioxidant may be combined. Pharmaceutically acceptable carriers and formulations suitable for the present disclosure are described in detail in Remington's Pharmaceutical Sciences, 19th ed., 1995.

In the present disclosure, the "individual" may be a mammal including humans, dogs, cats, cows, horses, pigs, mice, etc., but is not particularly limited thereto.

### [Advantageous Effects]

If the region to be removed by surgery is marked in advance, or if it is necessary to mark the location of the diseased lesion for a certain period of time, an image contrast agent is locally injected into the lesion, but as it spreads to surrounding tissues with diffusing at a fast rate, there is a disadvantage that the accuracy of image discrimination is deteriorated, and the image signal strength is rapidly weakened.

The composition for preparing an alginic acid-based injectable hydrogel containing an image contrast agent according to the present disclosure allows the contrast agent contained in the gel to remain at the injected lesion site for a certain period of time without spreading to the surroundings, thereby allowing the location of the target site to be determined relatively accurately with image and to be removed surgically, if necessary.

In addition, when the drug is injected intravenously, in most cases, it affects unwanted cells or organs, causing serious side effects in many cases. Even when the drug is injected locally at the target site, the drug is rapidly absorbed and spread at the injected site, so that side effects on the surrounding normal cells cannot be completely avoided, and the duration of the drug at the injection site is also shortened.

The composition for preparing an alginic acid-based injectable hydrogel containing a drug according to the present disclosure may be sustained for a long time in drug efficacy as the drug contained in the gel at the site of the injected lesion is slowly released for a certain period of time without being diffused to the surroundings. Therefore, even if a small amount of the drug is injected, the desired drug effect may be achieved at the target site, while reducing the side effects of the drug on the surrounding tissues.

### [Description of Drawings]

FIG. 1 is a configuration diagram of an alginic acid-based injectable hydrogel system.
FIG. 2A is a fluorescence graph according to the concentration of indocyanine green.
FIG. 2B is a graph showing the maximum fluorescence intensity according to the concentration of indocyanine green.
FIG. 2C is an absorbance graph according to the concentration of indocyanine green.
FIG. 3A is a fluorescence graph according to the concentration of indocyanin green-human serum albumin bound at 1:1.
FIG. 3B is a graph showing the maximum fluorescence intensity according to the concentration of indocyanine green-human serum albumin bound at 1:1.
FIG. 3C is an absorbance graph according to the concentration of indocyanin green-human serum albumin bound at 1:1.
FIG. 4A is a fluorescence graph according to the ratio of human serum albumin when the concentration of indocyanine green is fixed at 30 µM.
FIG. 4B is a graph showing the maximum fluorescence intensity according to the ratio of human serum albumin when the concentration of indocyanine green is fixed at 30 µM.
FIG. 4C is an absorbance graph according to the ratio of human serum albumin when the concentration of indocyanine green is fixed at 6 µM.
FIG. 5 is a photograph and graph showing the gelation time according to the concentration of calcium ions and D-gluconic acid in the alginic acid hydrogel.
FIG. 6A is a graph observing change in fluorescence intensity until 3 days after injecting the three conditions of alginic acid hydrogel containing 30 µM of indocyanine green solution, indocyanine green-human serum albumin solution, and 30 µM of indocyanine green-human serum albumin into 96 wells.
FIG. 6B is a fluorescence image taken immediately after injecting the three conditions of alginic acid hydrogel containing 30 µM of indocyanine green solution, indocyanine green-human serum albumin solution, and 30 µM of indocyanine green-human serum albumin into 96 wells.
Fig 7 is a schematic diagram of subcutaneous injection of a nude mouse by putting an alginic acid solution as the first agent wherein indocyanine green-human serum albumin and calcium carbonate are mixed, in one side of a dual syringe, and putting an alginic acid solution mixed with D-gluconic acid as the second agent in the other side.
FIG. 8 is a fluorescence image obtained over time for three conditions of an indocyanine green solution, an indocyanine green-human serum albumin solution, and an alginic acid hydrogel containing an indocyanine green-human serum albumin injected subcutaneously of a nude mouse.
FIG. 9 is a photograph observing an appearance of the injected site over time for three conditions of an indocyanine green solution, an indocyanine green-human serum albumin solution, and an alginic acid hydrogel containing an indocyanine green-human serum albumin injected subcutaneously to a nude mouse.
FIG. 10A is a graph representing a region of interest (ROI) value over time for three conditions of an indocyanine green solution, an indocyanine green-human serum albumin solution, and an alginic acid hydrogel containing an indocyanine green-human serum albumin injected subcutaneously to a nude mouse.
FIG. 10B is a graph representing TBR (target-to-background ratio = fluorescence ROI value at the labeling site ÷ fluorescence ROI value of the surrounding tissue) of the labeling site and the surrounding tissue over time for three conditions of an indocyanine green solution, an indocyanine green-human serum albumin solution, and an alginic acid hydrogel containing an indocyanine green-human serum albumin injected subcutaneously to a nude mouse.
FIG. 10C is a graph representing area of the spreading fluorescence at the labeling site over time for three conditions of an indocyanine green solution, an indocyanine green-human serum albumin solution, and an alginic acid hydrogel containing an indocyanine green-human serum albumin injected subcutaneously to a nude mouse.
FIG. 11A a structure of ZW800-1C NHS ester.
FIG. 11B is a graph of absorbance and fluorescence of covalently bound human serum albumin-ZW 800-1C conjugate.
FIG. 11C is a fluorescence image obtained over time for an alginic acid hydrogel containing a fluorescent dye in the form of a covalent bond of human serum albumin-ZW 800-1C injected subcutaneously to a nude mouse.
FIG. 12 is a view showing that doxorubicin, which is a drug, added to alginic acid hydrogel is injected into an ep-tube.
FIG. 13 is a fluorescence image obtained for distilled water, an alginic acid hydrogel, and alginic acid hydrogel containing doxorubicin.
FIG. 14 is a view showing that manganese chloride and ferridex, which is an MRI contrast agent, added to an alginic acid hydrogel are injected into an ep-tube.
FIG. 15 is an MRI T1 highlighted image obtained for distilled water, an alginic acid hydrogel, and an alginic acid hydrogel containing manganese chloride (0.05, 0.1, 0.2 mM) .
FIG. 16 is an MRI T2 highlighted image obtained for distilled water, an alginic acid hydrogel, and an alginic acid hydrogel containing ferridex (25, 50, 100 µM).
FIG. 17 is a view showing that Rose Bengal, which is a CT contrast agent, added to an alginic acid hydrogel, is injected into an ep-tube.
FIG. 18 is a CT image obtained for distilled water, an alginic acid hydrogel, and an alginic acid hydrogel containing Rose Bengal.
FIG. 19 is a CT image obtained for distilled water, an alginic acid hydrogel, and an alginic acid hydrogel containing Rose Bengal.
FIG. 20 is a view showing that a hydrogel made by mixing alginic acid and hyaluronic acid is injected into an ep-tube.
FIG. 21 is a fluorescence detection image confirming the site where the hydrogel was injected through a fluorescent laparoscope 3 days after injecting an alginic acid hydrogel containing an indocyanine green-human serum albumin into the submucosal layer in the stomach of test group 1 (pig #1). The fluorescent signal shown in the dotted circle is a fluorescent signal generated at the site where the hydrogel was injected.
FIG. 22 is a fluorescence detection image confirming the site where the hydrogel was injected through a fluorescent laparoscope 30 minutes after injecting an alginic acid hydrogel containing an indocyanine green-human serum albumin into the submucosal layer in the stomach of test group 2 (pig #2). The fluorescent signal shown in the dotted circle is a fluorescent signal generated at the site where the hydration gel was injected.
FIG. 23 is a fluorescence image obtained using a fluorescent laparoscope after incision of the stomach of a pig (top: pig #1, bottom: pig #2).
FIG. 24 is a microscopic observation photograph of PLGA microspheres loaded with celecoxib.
FIG. 25A is an absorption spectrum obtained by dissolving celecoxib in an acetonitrile solution at various concentrations.
FIG. 25B is a standard curve obtained by measuring the absorbance of a celecoxib (acetonitrile) solution in each of concentration at a wavelength of 251 nm.
FIG. 25C is an absorption spectrum measured by dissolving a celecoxib-loaded PLGA microsphere solution at a concentration of 0.04 mg/mL in an acetonitrile solution.
FIG. 26A is a fluorescence image obtained 24 hours, 48 hours, and 72 hours after injecting an alginic acid hydrogel containing PLGA microspheres loaded with celecoxib subcutaneously to a mouse.
FIG. 26B is a result for quantitatively analyzing the generated fluorescent signal over time at the injected site (region of interest, ROI) to which an alginic acid hydrogel is injected without celecoxib (Alg Gel) and an alginic acid hydrogel (microsphere/Alg Gel) containing celecoxib-loaded PLGA microspheres were injected.

### [Best Mode]

Hereinafter, preferred embodiments of the present disclosure will be described in more detail with reference to the accompanying drawings in order to describe the present disclosure in more detail. However, the present disclosure is not limited to the embodiments described herein and may be embodied in other forms. Unless otherwise defined, all technical and scientific terms used in this specification have the same meaning as commonly understood by an expert skilled in the art to which the present disclosure belongs. In general, the nomenclature used in this specification is well known and commonly used in the art.

### Example 1: Experiment of incorporating surgical marker to alginic acid hydrogel

### 1-1: Measurement of fluorescence and absorbance of indocyanine green and indocyanine green-human serum albumin complex

Before developing a surgical marker using indocyanine green and human serum albumin conjugate, in order to determine the appropriate concentration of indocyanine green and human serum albumin conjugate, the two substances were mixed by each concentration to observe change in fluorescence (λₑₓ=720 nm) and absorbance.

### <Preparation Example 1>

3 mL of distilled water was added to 1.5 mg of indocyanine green (ICG, purchased from Sigma-Aldrich) and dissolved by stirring to prepare a high concentration of indocyanine green solution. Distilled water was added to the high concentration of indocyanine green solution and mixed through stirring to prepared a 2 to 100 µM solution.

### <Preparation Example 2>

1 mL of the high concentration of indocyanine green solution was taken, added to 42.89 mg of human serum albumin (HSA, purchased from Sigma-Aldrich), and mixed with each other thoroughly by pipetting to obtain a high concentration of indocyanine green-human serum albumin complex. The high concentration of the indocyanine green-human serum albumin solution was added to distilled water and stirred to prepared a 2 to 100 µM of solution.

### <Evaluation 1-Evaluation of fluorescence properties>

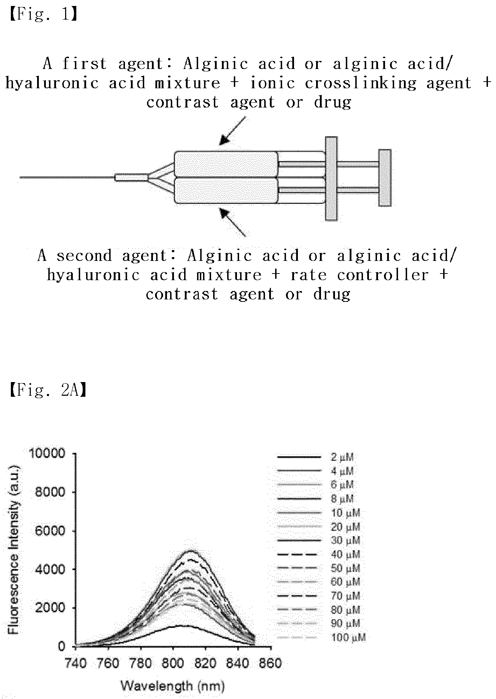

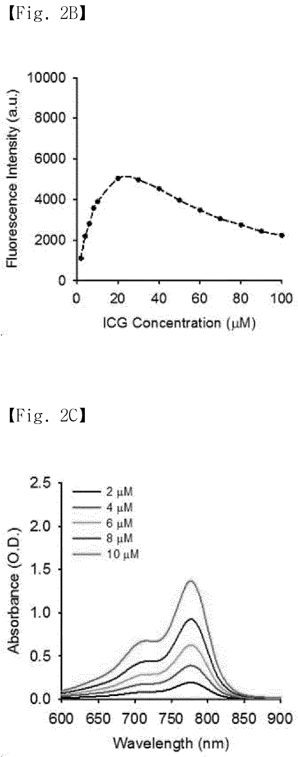

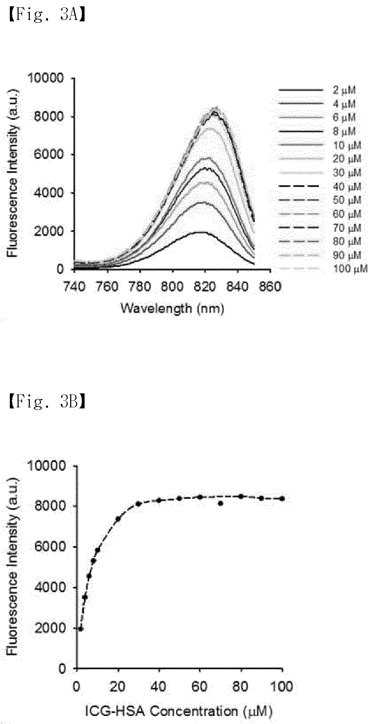

When only indocyanine green is in 2 to 100 µM, the maximum fluorescence intensity increased to about 20 µM, and then due to an aggregation of the fluorescent dye, the fluorescence decreased from the concentration or higher (FIGS. 2A and 2B). On the other hand, it was confirmed that the indocyanine green-human serum albumin complex bound in 1:1 had a higher fluorescence than the case of using only indocyanine green in the concentration range of 2 to 100 µM, and the maximum fluorescence intensity continued to increase until it became about 30 µM, and remained almost unchanged from the concentration or higher (FIGS. 3A and 3B).

### <Evaluation 2-Evaluation of absorbance properties>

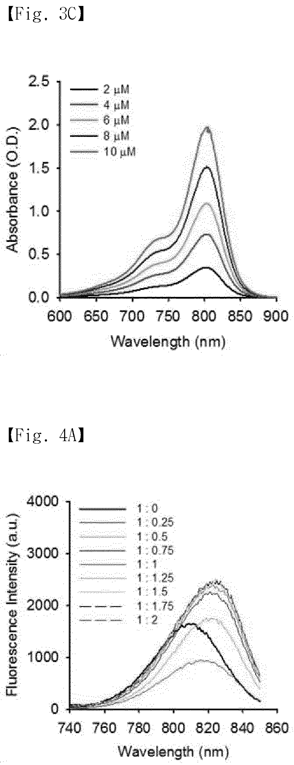

As a result of observing the absorbance of the indocyanine green and the indocyanine green-human serum albumin complex bound in 1:1 in concentration range of 2 to 10 µM, it was confirmed that both showed a tendency to increase the absorbance as the concentration increased, and the absorbance of the indocyanine green-human serum albumin complex appeared stronger (FIGS. 2C and 3C).

### 1-2: Measurement of fluorescence and absorbance according to mixing ratio of indocyanine green-human serum albumin complex

Before developing a surgical marker using indocyanine green-human serum albumin conjugate, in order to determine the appropriate mixed ratio of indocyanine green-human serum albumin conjugate, the two substances were mixed by each concentration to observe change in fluorescence (λₑₓ=720 nm) and absorbance.

### <Preparation Example 3>

In the same manner as in Preparation Example 1, a high concentration of indocyanine green solution was prepared. After adding 1 mL of distilled water to 42.89 mg of human serum albumin, pipetting was performed to thoroughly mix to prepare a high concentration of human serum albumin solution. The high concentration of indocyanine green solution was fixed to a final concentration of 30 µM by taking 0.125 mL, and only the human serum albumin solution was added in an amount corresponding to the molar ratio of indocyanine green of 0 to 2 to make a total of 1 mL of a solution with distilled water and stir to mix thoroughly.

### <Evaluation 3-Evaluation of fluorescence and absorbance properties>

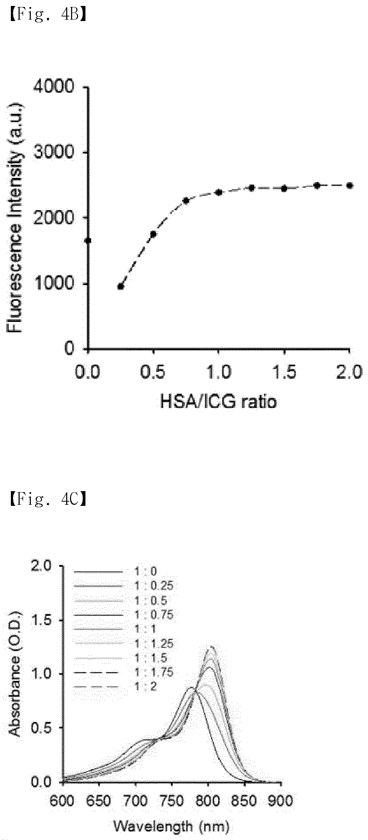

Changes in fluorescence and absorbance were observed using the solution of Preparation Example 3 (FIGS. 4A to 4C) . In the indocyanine green-human serum albumin conjugate, the fluorescence tended to increase as the amount of human serum albumin increased. In particular, fluorescence increased until the two substances were bound at a ratio of 1:1, and there was little change after the ratio of human serum albumin increased at or above that ratio. When looking at the absorbance according to the molar ratio of indocyanine green-human serum albumin conjugate, the absorbance tended to increase as the number of moles of human serum albumin increased.

### 1-3: Observation of gelation of alginic acid solution over time after addition of calcium

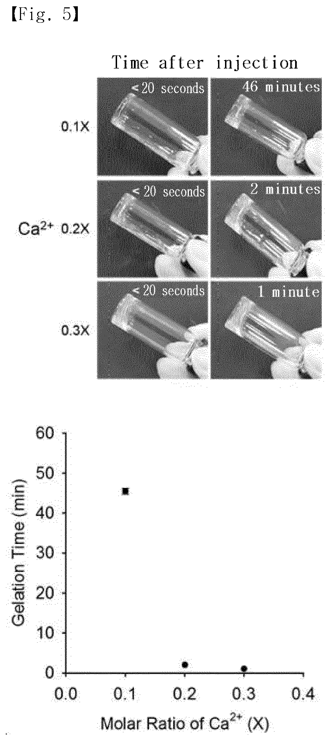

In order to make an alginic acid hydrogel with an appropriate gelation time that may be used as a surgical marker, calcium ions of different concentrations as shown in Table 1 were added to the alginic acid solution through a dual syringe, and then the change in the alginic acid solution over time was observed (FIG. 5).

Calcium ions form a hydrogel through cross-linking with alginic acid. Since calcium carbonate (purchased from Sigma-Aldrich) has low solubility, it was made to allow rapid cross-linking with alginic acid by regulating the dissolution rate of calcium carbonate with a mix of D-gluconic acid (purchased from Sigma-Aldrich), which is an acidic condition through a dual syringe. Through this process, alginic acid may be gelled evenly throughout. The pH of the total solution was adjusted to neutral by adding two folds of the number of moles of D-gluconic acid with respect to calcium carbonate.

**[Table 1]**

| Mixing condition | 1 | 2 | 3 |
|---|---|---|---|
| The first agent | Alginic acid 1.0 w/v% calcium carbonate 0.1X | Alginic acid 1.0 w/v% calcium carbonate 0.2X | Alginic acid 1.0 w/v% calcium carbonate 0.3X |
| The second agent | Alginic acid 1.0 w/v% D-gluconic acid 0.2X | Alginic acid 1.0 w/v% D-gluconic acid 0.4X | Alginic acid 1.0 w/v% D-gluconic acid 0.6X |

(The mixing condition of the alginic acid hydrogel through a dual syringe; X means the number of moles of the carboxy group contained in 1.0 w/v% of alginic acid.)

### <Preparation Example 4>

0.036 g of sodium alginate (purchased from Kimika) was dissolved in 3 mL of distilled water to prepare a 1.2 wt% solution of alginic acid. 0.25 mL of distilled water was added to 1.25 mL of a 1.2 wt% alginic acid solution to prepare a 1.0 wt% alginic acid solution. 1 mL of a 1.0 wt% alginic acid solution was added to 1.14 mg of calcium carbonate and mixed by pipetting or vortexing to prepare a first agent solution. 0.007 mL of a D-gluconic acid solution was added to 0.833 mL of 1.2 wt% of alginic acid solution, and made to a total of 1 mL with distilled water, followed by mixing completely through vortexing and stirring to prepare a second agent solution. Calcium carbonate and D-gluconic acid were used 0.1 and 0.2 times, respectively, based on the number of moles of carboxyl groups contained in 1.0 w/v% of alginic acid.

### <Preparation Example 5>

A solution was prepared in the same manner as in Preparation Example 4, except that the calcium carbonate of the first agent was 2.29 mg and the D-gluconic acid solution of the second agent was 0.015 mL. Calcium carbonate and D-gluconic acid were used 0.2 and 0.4 times, respectively, based on the number of moles of carboxyl groups contained in 1.0 w/v% of alginic acid.

### <Preparation Example 6>

A solution was prepared in the same manner as in Preparation Example 4, except that the calcium carbonate of the first agent was 3.43 mg and the D-gluconic acid solution of the second agent was 0.022 mL. Calcium carbonate and D-gluconic acid were used 0.3 and 0.6 times, respectively, based on the number of moles of carboxyl groups contained in 1.0 w/v% of alginic acid.

### <Evaluation 4-Evaluation of gelation of aqueous alginic acid solution according to amount of calcium ions>

After injecting the solution of the first agent and the second agent of Preparation Examples 4 to 6 to be a total volume of 0.5 mL in a glass bottle through a dual syringe, the formation of gel over time was observed by turning the glass bottle over. As shown in Table 1 and FIG. 5, an alginic acid solution was injected into a glass bottle with a dual syringe for a calcium condition of 0.1X to 0.3X, and the change of the solution over time was observed.

In the 0.1X calcium condition, the solution was in a liquid state (sol) flowing immediately after injection, and then changed to a solid state (gel) after about 46 minutes. In the 0.2X calcium condition, the solution was in a liquid state (sol) slightly flowing immediately after injection, and then changed to a solid state (gel) after about 2 minutes. Finally, in the 0.3X calcium condition, the solution exists in a state that hardly flow immediately after injection, and then changed to a solid state (gel) after about 1 minute. This gelation time is plotted on a graph according to the concentration of calcium ions used in FIG. 5.

It was observed that the gelation time of alginic acid decreased as the concentration of calcium ions added to alginic acid increased through 0.1 to 0.3X calcium conditions. Especially, among them, in the case of a calcium condition of 0.3X, it existed in a solid state (gel) that hardly flows immediately after injection. In this experiment, in order to effectively prevent the diffusion of the indocyanine green-human serum albumin complex, a subsequent experiment was conducted by selecting a calcium condition of 0.3X, which becomes a gel immediately after injection.

### 1-4: Fluorescence measurement of alginic acid hydrogel containing Indocyanine green-human serum albumin complex

In order to prevent the easy diffusion of indocyanine green in the living body, the indocyanine green-human serum albumin complex was incorporated into alginic acid, and then cross-linked with calcium ions through a dual syringe to form an injectable hydrogel, and fluorescence (λₑₓ=720 nm) and fluorescence images (λₑₓ=780 nm, λₑₘ=845 nm) were confirmed under in vitro conditions.

### <Preparation Example 7>

A high concentration of indocyanine green solution was prepared in the same manner as in Preparation Example 1. Distilled water was added to 0.139 mL of the high concentration of indocyanine green solution to make a total of 1.5 mL, followed by a mix with stirring to prepare the first agent solution. Distilled water was used as the second agent solution. The final solution of the first agent and the second agent was prepared so that the concentration of the indocyanine green was 30 µM.

### <Preparation Example 8>

A high concentration of indocyanine green-human serum albumin solution was prepared in the same manner as in Preparation Example 2. Distilled water was added to 0.139 mL of the high concentration of indocyanine green-human serum albumin to make a total of 1.5 mL, followed by a mix with stirring to prepare the first agent solution. Distilled water was used as the second agent solution. The final solution mixed with the first agent and the second agent was prepared so that the concentration of indocyanine green-human serum albumin complex was 30 µM.

### <Preparation Example 9>

A high concentration of indocyanine green-human serum albumin solution was prepared in the same manner as in Preparation Example 2. The first agent and the second agent solutions were prepared in the same method as in Preparation Example 6, except that 0.139 mL of the high concentration of indocyanine green-human serum albumin solution was added to 1.25 mL of a 1.2 wt% of alginic acid solution, and then the total volume was made to 1.5 mL with distilled water.

### <Evaluation 5-Fluorescence evaluation>

The solutions of Preparation Examples 7 to 9 were injected into 96 wells, respectively so that the total volume was 200 µL using a dual syringe. The fluorescence graph and fluorescence image of the injected gel were confirmed over time.

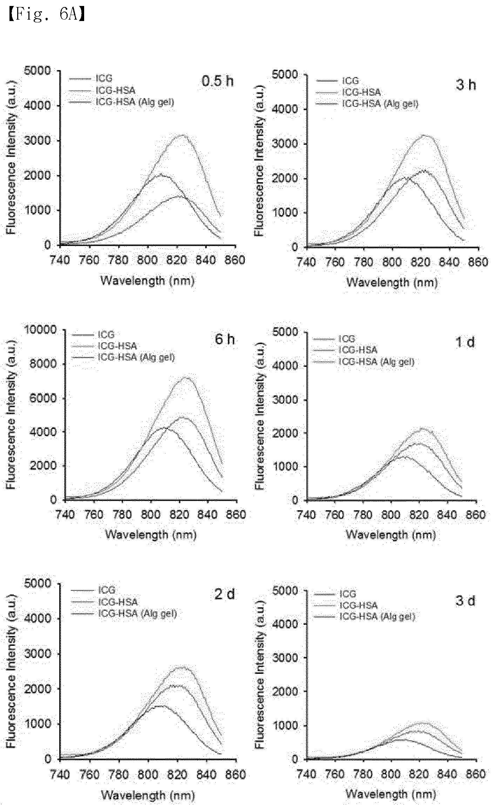

As shown in FIG. 6A, immediately after injection of each solution, the conditions of the indocyanine green-human serum albumin solution had the highest fluorescence, and the fluorescence was lowered in the order of the indocyanine green solution, alginic acid hydrogel containing indocyanine green-human serum albumin condition. 3 hours after injection, the fluorescence of the alginic acid hydrogel containing indocyanine green-human serum albumin was relatively high, and the fluorescence was lowered in the order of the indocyanine green-human serum albumin solution, alginic acid hydrogel containing indocyanine green-human serum albumin condition, and indocyanine green. The same results were also shown when these conditions were observed up to 3 days.

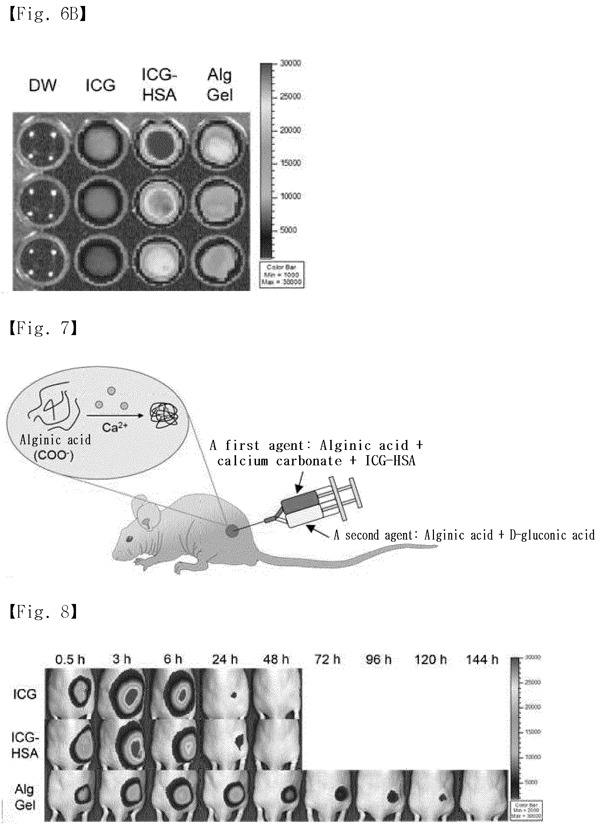

It was confirmed through the fluorescence image of FIG. 6B that the solution was well mixed and entered when the alginic acid hydrogel was injected with a dual syringe.

### 1-5: Fluorescence measurement of alginic acid hydrogel containing indocyanine green-human serum albumin in animal model

In order to see the fluorescence persistence of the alginic acid hydrogel containing indocyanine green-human serum albumin in a nude mouse, it was injected in the form of a gel subcutaneously to the nude mouse through a dual syringe, and then the labeling performance over time was photographed with a fluorescent image device (IVIS Lumina XR, Xenogen Corporation-Caliper, CA, USA). Three solutions to be injected into the animal model were prepared in the same manner as in Preparation Examples 7 to 9. FIG. 7 is a schematic diagram of injecting an alginic acid hydrogel containing indocyanine green-human serum albumin subcutaneously to a nude mouse using a dual syringe.

### <Evaluation 6-Evaluation of the persistence of fluorescence of gel in animal model>

The solutions of Preparation Examples 7 to 9 were injected subcutaneously to a nude mouse, respectively so that the total volume was 100 µL using a dual syringe.

FIG. 8 is a fluorescence image (λₑₓ=780 nm, λₑₘ=845 nm) obtained over time for three conditions of an indocyanine green solution, an indocyanine green-human serum albumin solution, and an alginic acid hydrogel containing an indocyanine green-human serum albumin injected subcutaneously to a nude mouse. At this time, it was confirmed that the fluorescence in the indocyanine green and indocyanine green-human serum albumin solution was maintained for only about one day, whereas in the case of the alginic acid hydrogel, fluorescence was continuously emitted for about 5 days. In addition, in the case of the alginic acid hydrogel, it was confirmed that the degree of the spreading of fluorescence dye was less than that of the other two conditions because the gel prevented the spreading of the fluorescence dye.

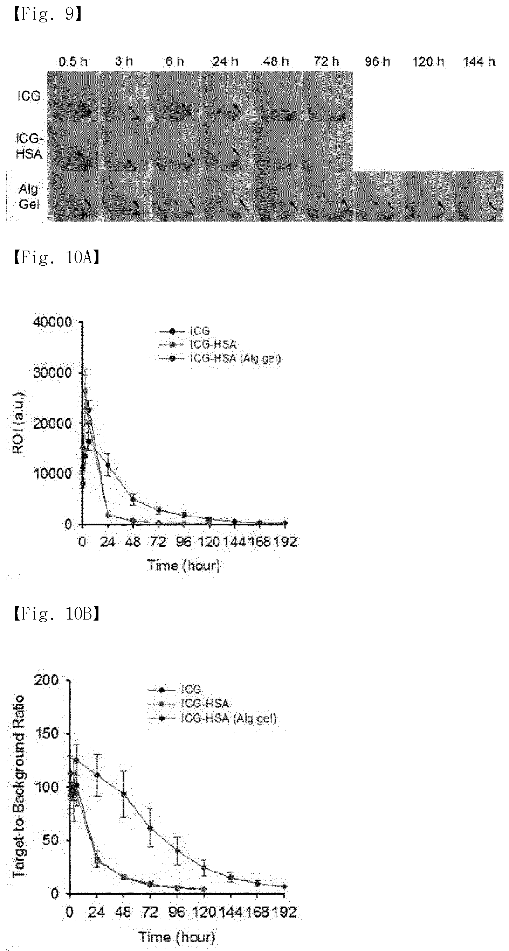

FIG. 9 is a result observing the injected site over time of an indocyanine green solution, an indocyanine green-human serum albumin solution, and an alginic acid hydrogel containing an indocyanine green-human serum albumin injected subcutaneously to a nude mouse. Specifically, it was confirmed that under the conditions of indocyanine green and indocyanine green-human serum albumin solution, the injection site appeared slightly convex immediately after injection, and after about 24 hours, the solution spreads and disappeared. On the other hand, in the case of alginic acid hydrogel, it was confirmed that the convex protruding region was clearly observed with the eye immediately after injection, and in the case of the gel, the decomposition was partly but maintained for about a month.

In FIG. 10A, the ROI value of the labeled region was compared to how long fluorescence of each condition persists in a nude mouse. When checking the ROI value, indocyanine green and indocyanine green-human serum albumin solutions showed almost similar patterns. It was confirmed that as time passed, the injected solution spreads and the fluorescence was greatest at 6 hours, and 16% and 12% of fluorescence remained, respectively, compared to immediately after injection when one day passed. On the other hand, in the case of the alginic acid hydrogel, it was confirmed that the fluorescence was the greatest at 6 hours as the solution spreads slightly after injection, but about 61% of the fluorescence signal still appeared compared to immediately after injection even after 2 days.

In FIG. 10B, a target-to-background ratio (TBR) was observed by dividing the fluorescence ROI value of the target site by the fluorescence ROI value of the surrounding tissue (background). As a result, it was confirmed that indocyanine green, indocyanine green-human serum albumin solution condition takes about 4 to 5 days for TBR to increase 5 times, whereas alginic acid hydrogel takes about 8 days for TBR to increase 5 times.

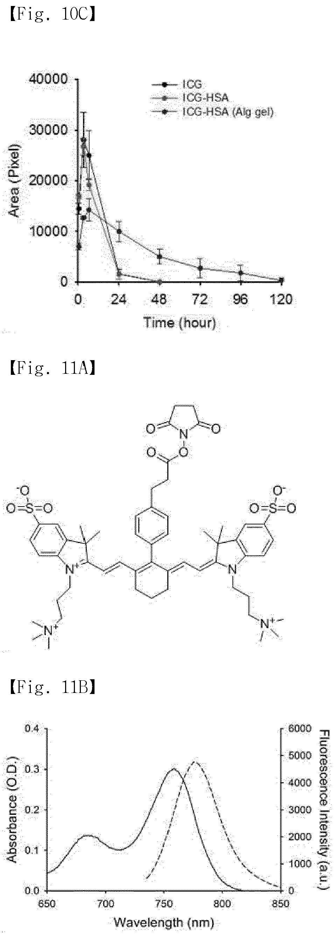

In FIG. 10C, the area where fluorescence appears in the nude mouse was confirmed. When comparing the area where the fluorescence spreads to the maximum in the conditions of indocyanine green and indocyanine green-human serum albumin solution and the area where the fluorescence spreads to the maximum in the alginic acid hydrogel, it can be seen that the fluorescence appears less diffuse about twice compared to that of the other conditions in the alginic acid hydrogel. In addition, the two conditions with different fluorescence signals almost disappeared in 48 hours, whereas the alginic acid hydrogel condition showed fluorescence signals up to 5 days.

Taking these results together, it was confirmed that the alginic acid hydrogel covered the fluorescent dye and prevented it from spreading at the target site under in vivo conditions, thereby effectively increasing the fluorescence duration.

### 1-6: Human serum albumin-ZW800-1C incorporation test of alginic acid hydrogel

Human serum albumin-ZW800-1C fluorescent dye, a covalently bound complex, was added to the alginic acid hydrogel to confirm the persistence of fluorescence under in vivo conditions.

### <Preparation Example 10>

Human serum albumin (10 mg/mL) was dissolved in a phosphate buffer, and then reacted with ZW800-1C NHS ester (FIG. 11A) in 1 mL of phosphate buffer at a molar ratio of 1:2 for 1 hour at room temperature. After completion of the reaction, unbound fluorescent dyes and reaction by-products were removed using a PD-10 column (GE Healthcare). The human serum albumin-ZW-800-1C conjugate obtained after purification was concentrated using an Amicon Ultra-0.5 mL (cut off: 30 kDa) centrifugal filter and stored in a refrigerator at 4°C.

In order to analyze the ratio of fluorescent dye bound to human serum albumin, human serum albumin-ZW800-1C conjugate was dissolved in a denatured phosphate buffer (including 1% SDS/1 mM NaOH) and absorbance was measured. The concentration of human serum albumin protein was calculated using the molar extinction coefficient (34,445 M^{- 1}cm⁻¹) of human serum albumin at 280 nm, and the fluorescent dye was used with a known molar extinction coefficient (125,000 M⁻¹cm⁻¹ at 779 nm in DMSO). As a result of the analysis, it was calculated that ZW800-1C was attached with 0.26 per one human serum albumin.

The degree of absorption and fluorescence of the human serum albumin-fluorescent dye conjugate was measured by diluting in a phosphate buffer at a concentration of 5 µM (based on the fluorescent dye concentration) (FIG. 11B).

### <Preparation Example 11>

The first agent and the second agent were prepared in the same manner as in Preparation Example 9, except that the fluorescent dye covalently bound to 30 µM of human serum albumin-ZW800-1C was used in place of the indocyanine green-human serum albumin complex in the first agent.

### <Evaluation 7-Evaluation of persistence of fluorescence of gel in animal model>

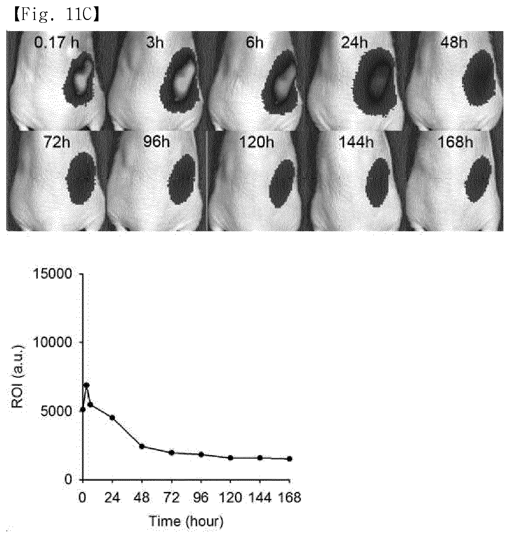

The solutions of Preparation Example 11 were injected subcutaneously to a nude mouse, so that the total volume was 100 µL using a dual syringe. In the in vivo condition, it was confirmed that even in the case of the fluorescent dye covalently bound to human serum albumin-ZW800-1C, the fluorescence duration was effectively increased by preventing diffusion at the target site (FIG. 11C).

### Example 2: Drug incorporation experiment of alginic acid hydrogel

Doxorubicin (purchased from Sigma-Aldrich) as an anticancer agent, was prepared as a drug to be incorporated into the alginic acid hydrogel.

### <Preparation Example 12>

1.5 mg of doxorubicin was dissolved in 0.5 mL of distilled water to prepare a high concentration of doxorubicin solution. The first agent and the second agent solutions were prepared in the same method as in Preparation Example 6, except that 0.125 mL of the high concentration of doxorubicin was added to 1.25 mL of a 1.2 wt% of alginic acid solution, and then the total volume was made to 1.5 mL with distilled water.

### <Evaluation 8-Doxorubicin incorporation in alginic acid hydrogel and fluorescence image confirmation>

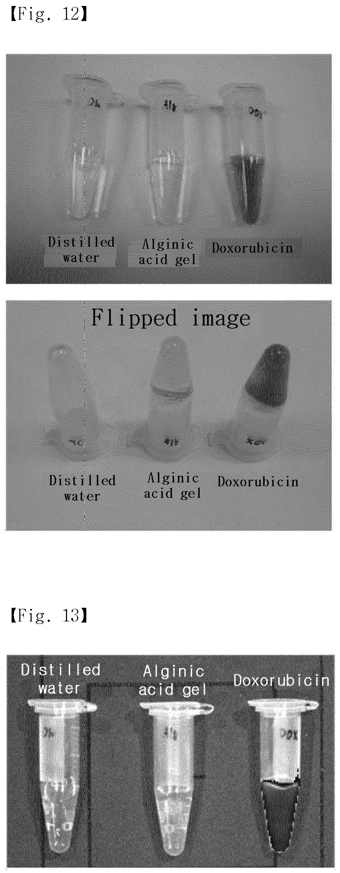

The solution of Preparation Example 12 was injected into the ep-tube so that the total volume was 0.5 mL using a dual syringe. Thereafter, it was confirmed that the alginic acid hydrogel formed by turning the ep-tube over as shown in FIG. 12 effectively contained doxorubicin.

As a result of examining the fluorescence image (λₑₓ=420 nm, λₑₘ=570 nm) of the hydrogel containing doxorubicin using a fluorescence image device (IVIS) as shown in FIG. 13, it was confirmed that doxorubicin was well spread throughout the gel.

### Example 3: Magnetic resonance image (MRI) contrast agent incorporation experiment of alginic acid hydrogel

A manganese chloride solution (manganese chloride, purchased from Sigma-Aldrich, 1M) and Ferdex I.V. (Feridex, purchased from Taejun Pharmaceutical co., Ltd., 11.2 mg Fe/mL) as the magnetic resonance image contrast agent were used as they are, as commercial products themselves are dispersed in aqueous solution.

### <Preparation Example 13>

A manganese chloride solution was diluted to 1/100 by adding 990 µL of distilled water to 10 µL of a manganese chloride solution. After adding 10, 20, and 40 µL of the diluted manganese chloride solution to 1.25 mL of a 1.2 wt% of alginic acid solution, respectively, the first agent and the second agent solutions were prepared in the same manner as in Preparation Example 6, except for using solutions of three concentrations (final concentrations 0.05, 0.1, 0.2 mM) made to 1.5 mL with distilled water.

### <Preparation Example 14>

Feridex solution was diluted to 1/20 by adding 950 µL of distilled water to 50 µL of Feridex solution. After adding 5, 10, and 20 µL of the diluted Feridex solution to 1.25 mL of a 1.2 wt% of alginic acid solution, respectively, the first agent and the second agent solutions were prepared in the same manner as in Preparation Example 6, except for using solutions of three concentrations (final concentrations 25, 50, 100 µM) made to 1.5 mL with distilled water.

### <Evaluation 9-Magnetic resonance image contrast agent incorporation in alginic acid hydrogel and magnetic resonance image confirmation>

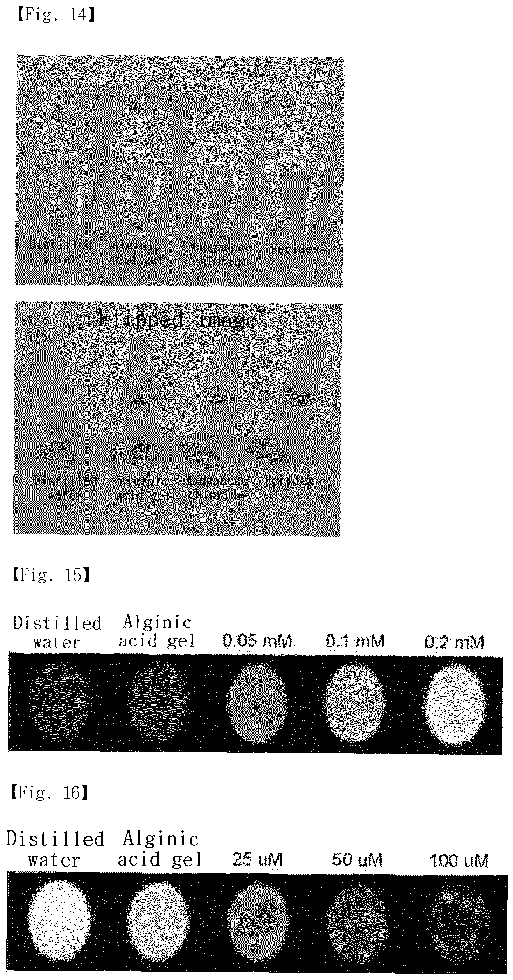

In the solutions of Preparation Examples 13 and 14, 0.2 mM of manganese chloride and 100 µM of Feridex solutions were injected into ep-tubes, respectively so that the total volume was 0.5 mL using a dual syringe. Thereafter, it was confirmed that the alginic acid hydrogel formed by turning the ep-tube over as shown in FIG. 14 effectively contained manganese chloride and Feridex which are magnetic resonance image contrast agent.

Three concentrations of the manganese chloride solutions of Preparation Example 13 were injected into the micro-tube so that the total volume was 0.25 mL using a dual syringe. Thereafter, as shown in FIG. 15, distilled water, alginic acid hydrogel, and manganese chloride (0.05, 0.1, 0.2 mM) were checked for MRI T1 highlighted images. It was confirmed that the MRI signal appeared brighter as the amount of manganese chloride added to the alginic acid hydrogel increased.

Three concentrations of the Feridex solutions of Preparation Example 14 were injected into the micro-tube so that the total volume was 0.25 mL using a dual syringe. Thereafter, as shown in FIG. 16, distilled water, alginic acid hydrogel, and Feridex (25, 50, 100 µM) were checked for MRI T2 highlighted images. It was confirmed that the MRI signal appeared brighter as the amount of Feridex added to the alginic acid hydrogel increased.

### Example 4: Computed tomography (CT) contrast agent incorporation experiment of alginic acid hydrogel

It was confirmed whether the CT contrast agent may be incorporated into the alginic acid hydrogel using Rose bengal (purchased from Sigma-Aldrich) as a CT contrast agent.

### <Preparation Example 15>

100 mg of Rose bengal was dissolved in 1 mL of distilled water to prepare a high concentration of Rose bengal solution. The first agent and the second agent solutions were prepared in the same manner as in Preparation Example 6, except for using a solution dissolved with stirring by adding 0.729 mL of a high concentration of Rose bengal and 0.771 mL of distilled water to 0.015 g of sodium alginate.

### <Evaluation 10-Rose bengal incorporation in alginic acid hydrogel and CT image conformation>

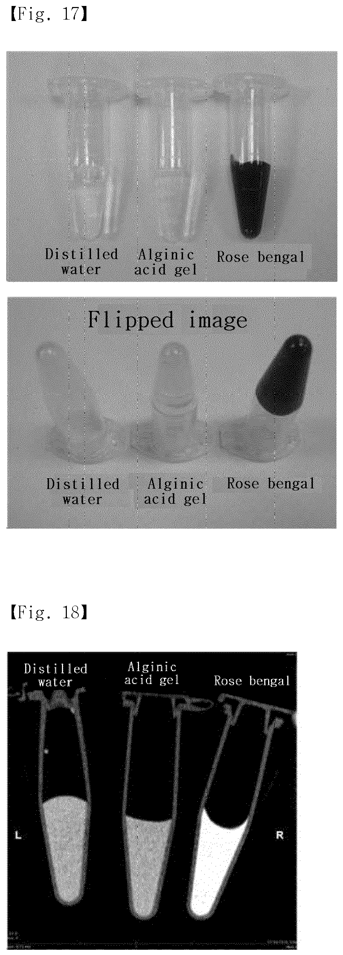

The solution of Preparation Example 15 was injected into the ep-tube so that the total volume was 0.5 mL using a dual syringe. Thereafter, it was confirmed that the alginic acid hydrogel formed by turning the ep-tube over as shown in FIG. 17 effectively contained Rose bengal.

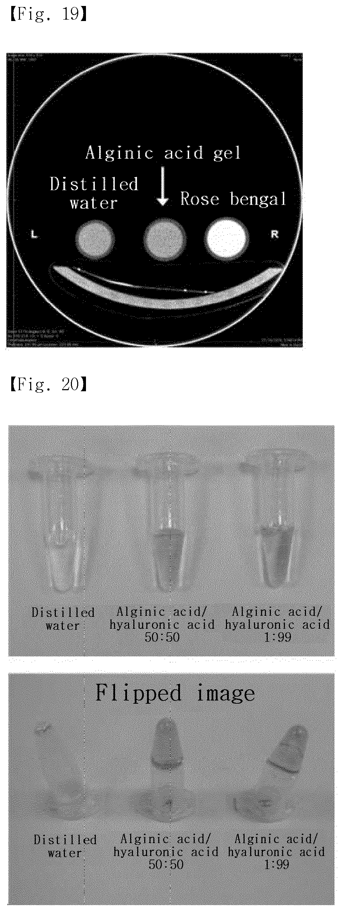

As shown in FIGS. 18 and 19, the CT images of the hydrogel containing distilled water, alginic acid hydrogel, and Rose bengal were confirmed. Through this, it was confirmed that Rose bengal was well spread throughout the alginic acid hydrogel.

### Example 5: Hydrogel experiment according to mixing ratio of alginic acid and hyaluronic acid

It was confirmed whether a hydrogel was formed under the conditions wherein alginic acid and hyaluronic acid were mixed at 50:50 and 1:99.

### <Preparation Example 16>

0.06 g of sodium hyaluronate (purchased from Lifecore Biomedical, Inc.) was dissolved in 2 mL of distilled water to prepare a 3 wt% of hyaluronic acid solution.

0.036 g of sodium alginate was dissolved in 3 mL of distilled water to prepare a 1.2 wt% of alginic acid solution. In the manner as in Preparation Example 1, a high concentration of indocyanine green solution was prepared.

0.334 mL of a 3 wt% of hyaluronic acid solution and 0.093 mL of a high concentration of indocyanine green solution were added to 0.417 mL of a 1.2 wt% of alginic acid solution, and the total volume was made 1 mL with distilled water.

1 mL of the mixed alginic acid and hyaluronic acid solution was added to 3.43 mg of calcium carbonate and vortexed to prepare the first agent solution. After adding 0.334 mL of a 3 wt% of hyaluronic acid solution and 0.022 mL of D-gluconic acid to 0.417 mL of a 1.2 wt% of alginic acid solution, the total volume was made 1 mL with distilled water, and then the solution of the second agent was prepared by mixing with vortexing and stirring.

### <Preparation Example 17>

In the same manner as in Preparation Example 16, a 3 wt% of hyaluronic acid solution, a 1.2 wt% of alginic acid solution, and a high concentration of indocyanine green solution were prepared.

0.66 mL of a 3 wt% of hyaluronic acid solution and 0.093 mL of a high concentration of indocyanine green solution were added to 0.005 mL of a 1.2 wt% of alginic acid solution, and the total volume was made 1 mL with distilled water. 1 mL of the mixed alginic acid and hyaluronic acid solution was added to 3.43 mg of calcium carbonate and vortexed to prepare the first agent solution.

After adding 0.66 mL of a 3 wt% of hyaluronic acid solution and 0.022 mL of D-gluconic acid to 0.005 mL of a 1.2 wt% of alginic acid solution, the total volume was made 1 mL with distilled water, and then the solution of the second agent was prepared by mixing with vortexing and stirring.

**[Table 2]**

| | Preparative example 16 | Preparative example 17 |
|---|---|---|
| Ratio of alginic acid and hyaluronic acid | 50:50 | 1:99 |

### <Evaluation 11-Evaluation of mixed hydrogel of alginic acid and hyaluronic acid>

The solution of Preparation Examples 16 and 17 was injected into the ep-tube, respectively so that the total volume was 0.5 mL using a dual syringe. Thereafter, as shown in FIG. 20, it was confirmed that the hydrogel was formed even under the condition of mixing alginic acid and hyaluronic acid formed by turning the ep-tube over.

### Example 6: Performance evaluation of alginic acid hydrogel containing indocyanine green-human serum albumin in pig model

Pigs (mini-pigs) have similar metabolic processes, organ structures, and sizes to those of the human body, making it easy to perform experimental treatment and followup using an endoscope. In order to confirm the fluorescent labeling performance of alginic acid hydrogel containing indocyanine green-human serum albumin, which is a fluorescent surgical marker in pigs, alginic acid hydrogel containing indocyanine green-human serum albumin was injected in the inner submucosal layer of the stomach of two pigs, and on the injected day and 3 days after the injection of the hydrogel, the fluorescent signal generated from the fluorescent dye injected into the stomach was detected as an image through a fluorescent laparoscope to evaluate whether the labeled location could be identified.

### <Preparation Example 18>

A high concentration of indocyanine green-human serum albumin solution was prepared in the same manner as in Preparation Example 2. After adding 0.558 mL of the high concentration of indocyanine green-human serum albumin solution to 8.4 mL of a 1 wt% of alginic acid solution, distilled water was further added to make the total volume to 12 mL, thereby preparing a fluorescent surgical marker solution. In this test, an experiment was conducted without adding an ionic crosslinking agent.

### <Evaluation 12-Confirmation of fluorescent labeling performance using fluorescent laparoscope on pigs>

Two pigs (pig #1, pig #2) were fasted for 48 hours before injecting the prepared alginic acid hydrogel, and the experiment was conducted after systemic anesthesia.

On the first day of the test, an endoscope and a catheter (Clear-Jet injector, 23G, Finemedix Co., Ltd.) were inserted through the mouth of test group 1 (pig #1), and the indocyanine green-human serum albumin-containing alginic acid hydrogel of Preparation Example 18 was injected by 1 mL into each of the three lower mucous layers in the pig's stomach. In order to confirm the injected location, the hemostasis clip was clipped right next to the hydrogel injected location. Three days after injection, a fluorescent laparoscopy system was installed in the pig's abdominal cavity, and the fluorescent signal generated from a fluorescent surgical marker located inside the stomach was detected through a fluorescent laparoscope located outside the stomach, and thereby it was evaluated whether it can be confirmed with real-time detection of the location where the surgical marker was labeled.

On the day of the fluorescent laparoscopy evaluation of test group 1, fluorescent surgical markers were injected into the three submucosal layers in the stomach in the same manner as performed in test group 1 for test group 2 (pig #2). In order to confirm the injected location, the hemostasis clip was clipped right next to the injected location. After injection of the hydrogel, a fluorescent laparoscopy system was installed in the pig's abdominal cavity, and the fluorescent signal generated from a fluorescent surgical marker located inside the stomach was detected through a fluorescent laparoscope located outside the stomach, and thereby it was evaluated whether it can be confirmed with real-time detection of the location where the surgical marker was labeled.

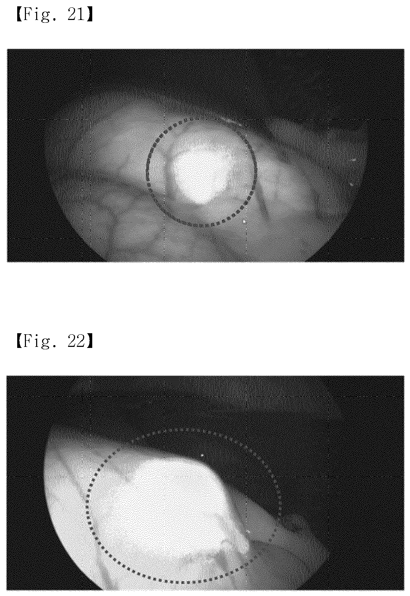

As a result of observing the location where alginic acid hydrogel containing indocyanine green-human serum albumin was injected, from an image using a fluorescent laparoscopic system, the locations where the hydration gel was injected could be confirmed from the fluorescence image both in test group 1 where a fluorescent marker was injected prior to 3 days (FIG. 21) and observed test group 2 where it was injected on the same day (FIG. 22).

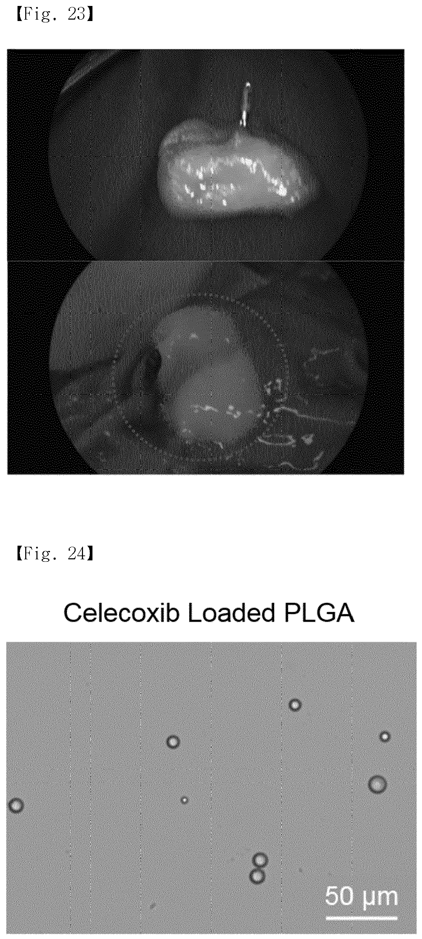

Subsequently, in order to confirm that the location identified from the fluorescence image was the location where the hydrogel was actually injected, the pig was euthanized and the pig's stomach was incised. It was confirmed that a fluorescent signal was generated next to the location where a haemostasis clip clipped right next to the location where the hydrogel was injected, and from this result, it was found that the fluorescence confirmed from the fluorescence laparoscopic image of the pig experiment was a fluorescence signal generated from the actual hydrogel (FIG. 23).

Taking these results together, it can be seen that the alginic acid gel containing indocyanine green-human serum albumin had excellent performance as an image marker for the area to be removed by surgery.

### Example 7: Image signal enhancement test in alginic acid hydrogel containing anti-inflammatory agent

When an alginic acid hydrogel is injected into the lower mucosa for image labeling, the patient's immune system recognizes that a foreign substance has invaded into the living body and may cause an inflammatory reaction in removing the foreign substance. At this time, proteins including various enzymes, complement systems, antibodies, and immune cells are introduced into the injection site, and actions to remove alginic acid and fluorescent dyes present at the injection site occur. Therefore, there is a possibility that the rate at which the fluorescent dye contained in the hydrogel is removed due to the inflammatory reaction occurring at the injection site would be fast.

Therefore, in the Example, biodegradable microspheres loaded with an anti-inflammatory agent was prepared and injected into a living body together with a hydrogel to suppress the inflammatory reaction at the injection site, and accordingly, a strength of the fluorescence image signal at the injected site was evaluated whether the strength could also be maintained higher. Celecoxib was used as an anti-inflammatory agent, and drug-loaded microspheres were prepared using a biodegradable polymer, poly(lactic-co-glycolic acid) (PLGA).

### 7-1. Preparation and analysis of biodegradable microspheres loaded with anti-inflammatory agents.

### <Preparation Example 19>

135 mg of PLGA (50:50, Mw 38,000-54,000, purchased from Sigma-Aldrich) and 13.5 mg of celecoxib (purchased from Sigma-Aldrich) were dissolved in 4.5 mL of dichloromethane. 0.5 g of polyvinyl alcohol (PVA, Mw 13,000-23,000, purchased from Sigma-Aldrich) was added to 50 mL of distilled water, heated to 65°C to dissolve completely, and then the solution was cooled at 25°C. A beaker containing an aqueous solution wherein polyvinyl alcohol as a surfactant is dissolved, was immersed in ice, and a dichloromethane solution wherein celecoxib and PLGA are dissolved, was added dropwise, while a homogenizer (Homogenizer PT 3100 Polytron, Kinematica AG, Littau-Lucerne, Switzerland) was stirred vigorously at 5,000 rpm for 5 minutes to prepare an oil-in-water emulsion solution. To remove the organic solvent, dichloromethane, the prepared emulsion solution was mixed with 100 mL of distilled water, and stirred at 300 rpm for 8 hours under a temperature condition of 25°C. Centrifugation and washing were repeated 3 times at 3,000 rpm for 5 minutes in order to remove PVA, which is a surfactant present on the surface of the prepared microspheres. Thereafter, the aqueous solution was freeze-dried, and the obtained microspheres were stored at 4°C.

### <Evaluation 13-Analysis of microspheres loaded with prepared anti-inflammatory agent>

After dispersing the PLGA microspheres loaded with celecoxib in distilled water at a concentration of 0.5 mg/mL, a few drops ware added on a slide glass and the shape and size was observed by using an optical microscope (Axio Imager M2 microscope, Zeiss, Germany) (FIG. 24). It was confirmed that the prepared microspheres had a round shape, and when the size of 100 microspheres was measured, it was confirmed that microspheres having a size of 9.4 ± 2.7 µm were prepared. The microspheres loaded with celecoxib were dispersed in distilled water at a concentration of 0.5 mg/mL, and the zeta potential measured using a Zetasizer (ZetasizerNanoZS, Malvern Instruments Worces-tershire, UK) was measured to - 20.7 mV.

### <Evaluation 14-Analysis of amount of drug loaded in microspheres>

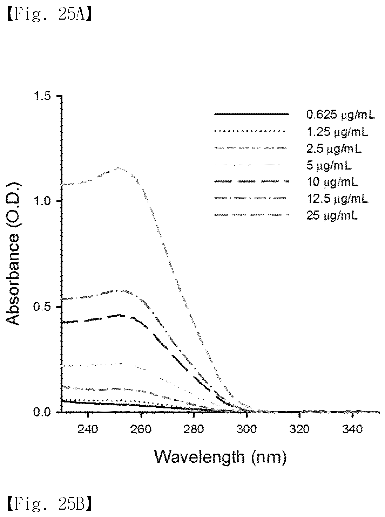

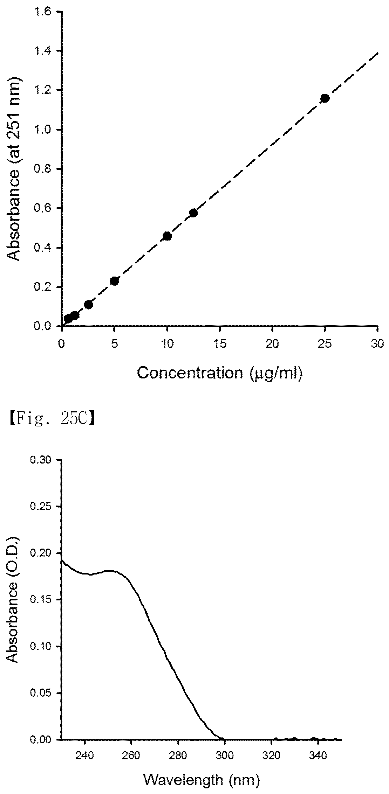

After dissolving 1 mg of celecoxib in 10 mL of acetonitrile, a standard solution of concentrations of 0.625, 1.25, 2.5, 5, 10, 12.5, and 25 µg/mL was prepared while diluting with acetonitrile. Each solution was measured for absorbance at a wavelength of 251 nm to obtain a standard curve for celecoxib (FIGS. 25A and 25B).

5 mg of PLGA microspheres loaded with celecoxib were completely dissolved in 2.5 mL of acetonitrile, diluted 50 times with acetonitrile solution, absorbance was measured at a wavelength of 251 nm, and the amount of drug contained in the microspheres was analyzed by comparison with the standard curve (FIG. 25C). As a result, it was confirmed that the amount of celecoxib contained in 2 mg of microspheres was 196.4 µg. That is, the drug was loaded with 9.8 wt/wt% in the microspheres.

### 7-2. Animal test evaluation

When alginic acid hydrogel containing indocyanine green-human serum albumin was mixed with celecoxib-loaded microspheres to suppress the inflammatory reaction, it was evaluated through animal experiments whether the fluorescent dye was maintained at the injected site for a longer time and the fluorescence intensity.

### <Preparation Example 20>

Indocyanine green-human serum albumin solution was prepared in the same manner as in Preparation Example 2. After adding 0.093 mL of an indocyanine green-human serum albumin solution to 1.167 mL of a 1.2 wt% of alginic acid solution, distilled water was further added to make the total volume of 2 mL, followed by stirring.

### <Preparation Example 21>

In order to prepare an alginic acid hydrogel containing an anti-inflammatory agent, 0.136 mL of a distilled water solution wherein PLGA microspheres loaded with celecoxib were dispersed at a concentration of 9 mg/mL was prepared. 2 mL of an alginic acid aqueous solution containing indocyanine green-human serum albumin was prepared as described above, and 0.136 mL of an aqueous microsphere solution and 2 mL of an alginic acid solution were mixed and stirred. The final concentration of celecoxib drug contained in the alginic acid hydrogel was 600 µg/mL.

### <Evaluation 15-Evaluation of persistence of fluorescence of gel in animal model>

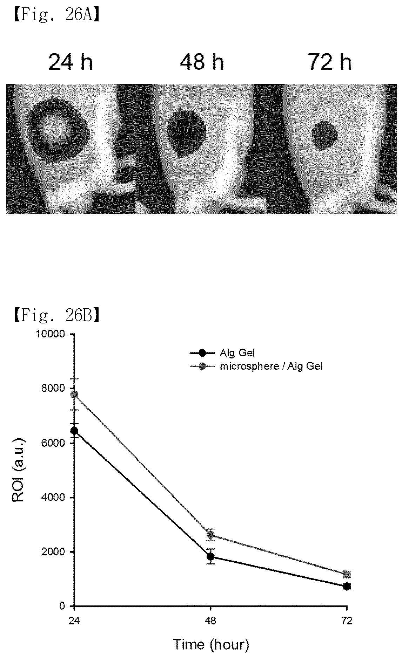

Using a syringe, each of 100 µL of the solutions of Preparation Examples 20 and 21 was subcutaneously injected to a white mouse. FIG. 26A is a fluorescence image (λₑₓ = 780 nm, λₑₘ = 845 nm) obtained at 24 hours, 48 hours, and 72 hours after injecting alginic acid hydrogel into a subcutaneous location of the mouse. When the fluorescence intensity generated at the region of interest (ROI) to which the hydrogel was injected was quantitatively analyzed for each time period, the fluorescence intensity which is 1.2 times higher at 24 hours, 1.4 times higher at 48 hours, and 1.6 times higher at 72 hours compared to the hydrogel without microspheres was detected (FIG 26B). This indicates that the inflammatory reaction at the site where the hydrogel was injected was suppressed due to the action of celecoxib, an anti-inflammatory drug loaded in the microspheres, and thereby that the fluorescent dye contained in the hydrogel was maintained at a higher concentration at the injected site.

## Claims

1. A composition for preparing an injectable hydrogel, comprising a first agent comprising aqueous alginic acid and an ionic crosslinking agent; and a second agent comprising aqueous alginic acid and a crosslinking rate controller,
wherein the crosslinking rate controller comprises any of one or more selected from the group consisting of D-gluconic acid, acetic acid, malic acid, lactic acid and ascorbic acid.

2. The composition of claim 1, wherein the ionic crosslinking agent is selected from the group consisting of calcium carbonate, strontium carbonate, calcium disodium edetate (calcium EDTA), calcium citrate, calcium sulfate, calcium alginate, calcium gluconate, calcium phosphate dibasic (CaHPO4), barium carbonate, zinc carbonate, calcium chloride, calcium lactate, calcium aspartate, calcium saccharate, calcium oxovalerate, calcium lactobionate, calcium lactogluoconate, and a mixture thereof.

3. The composition of claim 1, wherein the first agent or the second agent comprises an image contrast agent or a drug.

4. The composition of claim 1, wherein the alginic acid is mixed with hyaluronic acid.

5. The composition of claim 3, wherein the image contrast agent is a magnetic resonance image contrast agent, a CT contrast agent, or a fluorescent dye.

6. The composition of claim 5, wherein the fluorescent dye is the complex of human serum albumin and a fluorescent dye connected through an ionic bond, a hydrophobic bond or a covalent bond.

7. The composition of claim 3, wherein the drug is an anticancer agent, an anti-inflammatory agent, an anesthetic agent, or an immunotherapeutic agent.

8. The composition of claim 7, wherein the anti-inflammatory agent is biodegradable microspheres, nanoparticles, liposome or a form mounted on micelle.

9. The composition of claim 8, wherein the anti-inflammatory agent reduces the removal rate of the fluorescent dye by suppressing the inflammatory reaction at the injection site.

10. The composition according to any one of claims 1 to 9 for use in surgery.

11. The composition for use according to claim 10, wherein the use comprises labeling a lesion with the composition.

12. The composition according to any one of claims 1 to 9 for use as a medicament, wherein the composition comprises a drug.

13. The composition for use according to claim 12, wherein the drug is selected from anticancer agents, anti-inflammatory agents, anesthetic agents, antiviral agents, antibacterial agents, therapeutic antibodies, antibiotics, or immunotherapeutic agents.

## Patentansprüche

1. Zusammensetzung zur Herstellung eines injizierbaren Hydrogels, umfassend ein erstes Mittel, das wässrige Alginsäure und einen ionischen Vernetzer umfasst; und ein zweites Mittel, das wässrige Alginsäure und einen Vernetzungsgeschwindigkeitsregler umfasst,
wobei der Vernetzungsgeschwindigkeitsregler eine von einer oder mehreren umfasst, die aus der Gruppe bestehend aus D-Gluconsäure, Essigsäure, Äpfelsäure, Milchsäure und Ascorbinsäure ausgewählt sind.

2. Zusammensetzung nach Anspruch 1, wobei der ionische Vernetzer aus der Gruppe bestehend aus Calciumcarbonat, Strontiumcarbonat, Calciumdinatriumedetat (Calcium-EDTA), Calciumcitrat, Calciumsulfat, Calciumalginat, Calciumgluconat, Calciumhydrogenphosphat (CaHPO4), Bariumcarbonat, Zinkcarbonat, Calciumchlorid, Calciumlactat, Calciumaspartat, Calciumsaccharat, Calciumoxovalerat, Calciumlactobionat, Calciumlactogluoconat und einem Gemisch davon ausgewählt sind.

3. Zusammensetzung nach Anspruch 1, wobei das erste Mittel oder das zweite Mittel ein Bildkontrastmittel oder einen Arzneistoff umfasst.

4. Zusammensetzung nach Anspruch 1, wobei die Alginsäure mit Hyaluronsäure gemischt ist.

5. Zusammensetzung nach Anspruch 3, wobei es sich bei dem Bildkontrastmittel um ein Magnetresonanzbildkontrastmittel, ein CT-Kontrastmittel oder einen Fluoreszenzfarbstoff handelt.

6. Zusammensetzung nach Anspruch 5, wobei es sich bei dem Fluoreszenzfarbstoff um den Komplex von Humanserumalbumin und einem Fluoreszenzfarbstoff, der über eine lonenbindung, eine hydrophobe Bindung oder eine kovalente Bindung verbunden ist, handelt.

7. Zusammensetzung nach Anspruch 3, wobei es sich bei dem Arzneistoff um ein Antikrebsmittel, einen Entzündungshemmer, ein Anästhetikum oder ein Immuntherapeutikum handelt.

8. Zusammensetzung nach Anspruch 7, wobei es sich bei dem Entzündungshemmer um biologisch abbaubare Mikrokügelchen, Nanopartikel, Liposom oder eine mizellgeträgerte Form handelt.

9. Zusammensetzung nach Anspruch 8, wobei der Entzündungshemmer die Abbaugeschwindigkeit des Fluoreszenzfarbstoffs durch Unterdrücken der Entzündungsreaktion an der Injektionsstelle verringert.

10. Zusammensetzung gemäß einem der Ansprüche 1 bis 9 zur chirurgischen Verwendung.

11. Zusammensetzung zur Verwendung gemäß Anspruch 10, wobei die Verwendung Markieren einer Läsion mit der Zusammensetzung umfasst.

12. Zusammensetzung gemäß einem der Ansprüche 1 bis 9 zur Verwendung als Medikament, wobei die Zusammensetzung einen Arzneistoff umfasst.

13. Zusammensetzung zur Verwendung gemäß Anspruch 12, wobei der Arzneistoff aus Antikrebsmitteln, Entzündungshemmern, Anästhetika, antiviralen Mitteln, antibakteriellen Mitteln, therapeutischen Antikörpern, Antibiotika oder Immuntherapeutika ausgewählt ist.

## Revendications

1. Composition pour la préparation d'un hydrogel injectable, comprenant un premier agent comprenant de l'acide alginique aqueux et un agent de réticulation ionique ; et un second agent comprenant de l'acide alginique aqueux et un régulateur de vitesse de réticulation,
dans laquelle le régulateur de vitesse de réticulation comprend l'un quelconque parmi un ou plusieurs choisis dans le groupe constitué par l'acide D-gluconique, l'acide acétique, l'acide malique, l'acide lactique et l'acide ascorbique.

2. Composition de la revendication 1, dans laquelle l'agent de réticulation ionique est choisi dans le groupe constitué par le carbonate de calcium, le carbonate de strontium, l'édétate de calcium disodique (EDTA de calcium), le citrate de calcium, le sulfate de calcium, l'alginate de calcium, le gluconate de calcium, le phosphate de calcium dibasique (CaHP04), le carbonate de baryum, le carbonate de zinc, le chlorure de calcium, le lactate de calcium, l'aspartate de calcium, le saccharate de calcium, l'oxovalérate de calcium, le lactobionate de calcium, le lactogluoconate de calcium et un mélange de ceuxci.

3. Composition de la revendication 1, dans laquelle le premier agent ou le second agent comprend un agent de contraste d'image ou un médicament.

4. Composition de la revendication 1, dans laquelle l'acide alginique est mélangé à de l'acide hyaluronique.

5. Composition de la revendication 3, dans laquelle l'agent de contraste d'image est un agent de contraste d'image par résonance magnétique, un agent de contraste CT ou un colorant fluorescent.

6. Composition de la revendication 5, dans laquelle le colorant fluorescent est le complexe de l'albumine sérique humaine et d'un colorant fluorescent reliés par une liaison ionique, une liaison hydrophobe ou une liaison covalente.

7. Composition de la revendication 3, dans laquelle le médicament est un agent anticancéreux, un agent anti-inflammatoire, un agent anesthésique ou un agent immunothérapeutique.

8. Composition de la revendication 7, dans laquelle l'agent anti-inflammatoire représente des microsphères biodégradables, des nanoparticules, un liposome ou une forme montée sur micelle.

9. Composition de la revendication 8, dans laquelle l'agent anti-inflammatoire réduit le taux d'élimination du colorant fluorescent en supprimant la réaction inflammatoire au site d'injection.

10. Composition selon l'une quelconque des revendications 1 à 9 pour une utilisation en chirurgie.

11. Composition pour une utilisation selon la revendication 10, dans laquelle l'utilisation comprend le marquage d'une lésion avec la composition.

12. Composition selon l'une quelconque des revendications 1 à 9 pour une utilisation comme médicament, dans laquelle la composition comprend un médicament.

13. Composition pour une utilisation selon la revendication 12, dans laquelle le médicament est choisi parmi des agents anticancéreux, des agents anti-inflammatoires, des agents anesthésiques, des agents antiviraux, des agents antibactériens, des anticorps thérapeutiques, des antibiotiques ou des agents immunothérapeutiques.
